(19) Europäisches Patentamt
European Patent Office
Office européen des brevets

(11) **EP 2 916 119 B1**

## (12) EUROPÄISCHE PATENTSCHRIFT

(45) Veröffentlichungstag und Bekanntmachung des Hinweises auf die Patenterteilung:
**30.11.2016   Patentblatt 2016/48**

(51) Int Cl.:
*G01K 1/20* (2006.01)        *G01K 7/42* (2006.01)

(21) Anmeldenummer: **15000498.4**

(22) Anmeldetag: **20.02.2015**

(54) **MESSVORRICHTUNG UND MESSVERFAHREN ZUR ERFASSUNG EINER UMGEBUNGSTEMPERATUR EINES MEDIZINISCHEN GERÄTS SOWIE VORRICHTUNG UND VERFAHREN ZUR MEDIZINISCHEN INSUFFLATION**

MEASURING DEVICE AND MEASURING METHOD FOR DETECTING AN AMBIENT TEMPERATURE OF A MEDICAL DEVICE AND DEVICE AND METHOD FOR MEDICAL INSUFFLATION

DISPOSITIF DE MESURE ET PROCÉDÉ DE MESURE DESTINÉ À DÉTECTER UNE TEMPÉRATURE AMBIANTE D'UN APPAREIL MÉDICAL ET DISPOSITIF ET PROCÉDÉ D'INSUFFLATION MÉDICALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.03.2014   DE 102014002762**

(43) Veröffentlichungstag der Anmeldung:
**09.09.2015   Patentblatt 2015/37**

(73) Patentinhaber: **Storz Endoskop Produktions GmbH**
**78532 Tuttlingen (DE)**

(72) Erfinder:
• **Hauser, Walter**
  **CH-8200 Schafthausen (CH)**
• **Schmidt, Manfred**
  **D-78476 Allensbach (DE)**
• **Stillhard, Otmar**
  **CH-8266 Steckborn (CH)**

(74) Vertreter: **Fugmann, Winfried**
**Ebeneweg 4**
**78333 Stockach (DE)**

(56) Entgegenhaltungen:
DE-A1-102006 019 402     US-A- 4 741 476
US-A1- 2005 209 813     US-A1- 2012 277 673

## Beschreibung

Die vorliegende Erfindung betrifft eine Messvorrichtung und ein Messverfahren zur Erfassung einer Umgebungstemperatur eines Wärme abgebenden oder seiner Umgebung Wärme entziehenden medizinischen Geräts sowie eine Vorrichtung und ein Verfahren zur medizinischen Insufflation.

Aus EP 0 564 953 A1 ist es bekannt, bei einer Insufflation von $CO_2$- oder $N_2O$-Gas in einen biologischen Körper einen mit einer Heizdrahtwicklung versehenen Heizschlauch zu verwenden, durch den das Gas zu einem Insufflationsinstrument geführt und dabei etwa auf die Körpertemperatur des Patienten erwärmt wird. Im distalen Bereich des Heizschlauchs ist ein Temperaturfühler integriert. Die Temperatur eines dem Körper zugeführten Gasvolumens, die von dem Temperatursensor erfasst worden ist, kann durch die im Heizschlauch vorgesehene Heizung nicht mehr verändert werden, sondern lediglich die Temperatur eines nachfolgend zugeführten Gasvolumens.

Gemäß DE 10 2006 019 402 A1 wird bei einem Gerät zur transnasalen Insufflation (TNI) das einem Patienten zugeführte Gas auf eine Temperatur erwärmt, die etwa 10 K über der Umgebungstemperatur liegt. In Abhängigkeit von der von einem Umgebungstemperatursensor erfassten Umgebungstemperatur wird eine Sollgastemperatur des Gases am Auslass eines Befeuchters bestimmt und eine Heizleistung der Befeuchterheizung gesteuert, so dass eine Istgastemperatur möglichst gleich der Sollgastemperatur ist.

Gemäß DE 692 23 723 T2 wird bei einem Atemluftbefeuchter mit einem Umgebungstemperaturfühler die Umgebungstemperatur der umgebenden Atmosphäre erfasst und in Abhängigkeit von der Umgebungstemperatur eine Gasauslasstemperatur berechnet, um zur Verminderung der Kondensation des befeuchteten Gases Heizmittel selektiv mit Energie zu versorgen.

Aus DE 10 2005 049 676 B3 ist ein Verfahren zur berührungslosen Bestimmung der Körperkerntemperatur eines Menschen bekannt, wobei die Oberflächentemperatur des Menschen an einem Körpermessort mittels einer beabstandet zu demselben angeordneten Temperatursensoreinheit erfasst wird und das Sensorsignal einer Auswerteeinheit übergeben wird. Unter Berücksichtigung der Differenz zwischen der Oberflächentemperatur und einer Umgebungstemperatur wird auf die Körperkerntemperatur geschlossen, wobei die Umgebungstemperatur durch eine Stabilisierungsvorrichtung auf eine vorgegebene Temperatur geregelt bzw. stabilisiert wird. Nach WO 2010/023255 A1 wird eine Temperatur eines Körpers mit Hilfe eines ersten und eines zweiten Temperatursensors gemessen, die in einem Material eingeschlossen sind, wobei eine Kontaktfläche den Körper kontaktiert und der erste und der zweite Temperatursensor in unterschiedlicher Höhe über der Kontaktfläche angeordnet sind. Eine genaue Erfassung einer Umgebungstemperatur erfolgt bei den vorgenannten Verfahren nicht.

Die gattungsfremde DE 198 00 753 A1 offenbart einen Sensor für eine nicht-invasive Temperaturmessung eines Fluids, das in einem Rohr strömt, wobei aus dem Wärmeflussverhalten des Fluids und extern gemessenen Temperaturen die zu messende Temperatur des Fluids ermittelt wird. In der ebenfalls gattungsfremden DD 87 677 B1 ist eine Vorrichtung zur Messung der Guttemperatur flüssiger und zähviskoser Massen mit einem stabförmigen Temperaturfühler beschrieben, der in das zu messende Gut hineinragt und an einen Thermostat, einen Verstärker und an ein Anzeige- oder Registriergerät angeschlossen ist. Dabei ist eine Messstelle an der Spitze und eine am Schaft des Temperaturfühlers vorgesehen. Eine korrigierte Thermospannung wird dadurch ermittelt, dass die mit einer von der Konstruktion und dem Einbau des Fühlers sowie dem zu messenden Gut abhängigen Konstante multiplizierte Differenz der Thermospannungen zur Thermospannung der Messstelle an der Fühlerspitze addiert wird. Zur Ausschaltung des Einflusses der Umgebungstemperatur wird ein Thermostat verwendet. Ferner ist in der gattungsfremden GB 2 131 175 A eine in die Wand einer Brennkammer eingesetzte Sonde offenbart, die zwei voneinander getrennte längliche Körper umfasst, die jeweils einen distalen und einen proximalen Temperaturfühler enthalten. Das proximale Ende der Sonde kann mittels einer Kühlflüssigkeit auf einer kontrollierten Temperatur gehalten werden. Auch bei den vorgenannten Vorrichtungen wird keine Umgebungstemperatur ermittelt.

In US 2012/277673 A1 ist eine Bluttemperatursensoranordnung für ein Gerät zur Hämodialyse offenbart, bei dem Blut durch ein Schlauchset gepumpt wird. Die Temperatur des Bluts wird mit einem nicht-invasiven Bluttemperatursensor erfasst. An einem Temperatursensorgehäuse ist ein Umgebungstemperatursensor angebracht, mit dem die Umgebungstemperatur gemessen und dazu benutzt wird, um die Bluttemperaturmessung zu korrigieren. Die Temperatur des Bluts wird durch Regelung der Temperatur des Dialysats in einem gewünschten Bereich gehalten.

Gemäß US 2005/0209813 A1 umfasst eine Temperaturmessvorrichtung einen ersten und einen zweiten Temperatursensor, die in einem ersten bzw. in einem zweiten Abstand zu einer Wand eines Raums oder Gebäudes an dieser angebracht werden können. Weiterhin ist ein Prozessor vorgesehen zur Abschätzung einer dritten Temperatur aufgrund der ersten und der zweiten Temperatur und des Abstands zwischen dem ersten und dem zweiten Temperatursensor. Dabei wird die Differenz der von den beiden Temperatursensoren erfassten Temperaturen verwendet.

Aus US 4,741,476 ist ein elektronischer Thermostat bekannt, der eine Korrektur für die Eigenwärme aufweist, die durch ein Halbleiterschaltelement erzeugt wird, das zum Einschalten eines Temperaturänderungsapparats dient. Dabei ist ein Halbleiter-Leistungsschaltelement in einem oberen Bereich einer vertikal orientierten Leiterplatte angeordnet, ein erster Thermistor ist in einem unteren Bereich der Leiterplatte und ein zweiter Thermistor in einem dazwischenliegenden Bereich angeordnet. Eine korrigierte Umgebungstemperatur wird aus der vom ersten Thermistor ermittelten Temperatur und der Differenz zwischen den vom ersten und vom zweiten Thermistor ermittelten Temperaturen erzeugt.

In vielen medizinischen Anwendungsfällen, etwa bei der Gasinsufflation, ist eine möglichst unverfälschte Erfassung der Umgebungstemperatur vorteilhaft. Es sind Temperatursensoren unterschiedlicher Art bekannt, die beispielsweise mit temperaturabhängigen Widerständen oder Kontaktspannungen arbeiten oder die als Halbleitersensoren oder als Infrarot-Strahlungssensoren ausgebildet sind. Wird ein derartiger Sensor zur Messung einer Lufttemperatur bzw. zur Messung der Temperatur in der Umgebung eines Gerätes eingesetzt, so ist dafür Sorge zu tragen, dass der von dem Sensor erfasste Temperaturwert nicht von einer durch eine Halterung des Sensors übertragene Wärmeeinwirkung bzw. durch die von dem Gerät beim Betrieb abgegebene Wärme verfälscht wird. Eine derartige Verfälschung tritt insbesondere dann ein, wenn der zur Erfassung der Umgebungstemperatur eingesetzte Temperatursensor an dem Gerät selbst angeordnet ist. Andererseits ist eine Anordnung eines Umgebungstemperatursensors in größerem Abstand von dem Gerät, zu dessen Steuerung die von dem Sensor ermittelte Umgebungstemperatur verwendet wird, umständlich, mit entsprechenden Leitungen bzw. Signalübertragungswegen verbunden und fehlerträchtig. Selbst wenn auf diese Weise eine Verfälschung des erfassten Temperaturwerts durch die von dem Gerät erzeugte Wärme vermieden werden kann, ist es dennoch nicht sichergestellt, dass die in größerem Abstand von dem Gerät gemessene Temperatur für die Umgebungstemperatur in einem Bereich, der für die Steuerung des Geräts relevant ist, tatsächlich repräsentativ ist.

Es ist Aufgabe der vorliegenden Erfindung, eine Messvorrichtung und ein Messverfahren zur Erfassung einer Umgebungstemperatur eines Wärme abgebenden oder seiner Umgebung Wärme entziehenden medizinischen Geräts anzugeben, wobei die oben genannten Nachteile möglichst vermieden werden. Insbesondere ist es Aufgabe der vorliegenden Erfindung, eine Messvorrichtung und ein Messverfahren zur Erfassung der Umgebungstemperatur anzugeben, wobei die Messvorrichtung an einem Wärme erzeugenden medizinischen Gerät angeordnet ist. Ferner ist es Aufgabe der Erfindung, eine Vorrichtung und ein Verfahren zur medizinischen Insufflation anzugeben, die bzw. das insbesondere hinsichtlich der Temperierung des einem Patienten zugeführten Gases verbessert ist.

Diese Aufgabe wird durch eine Messvorrichtung gemäß Anspruch 1, durch ein Messverfahren gemäß Anspruch 12 sowie durch eine Vorrichtung zur medizinischen Insufflation gemäß Anspruch 14 und durch ein Verfahren zur medizinischen Insufflation gemäß Anspruch 16 gelöst.

Vorteilhafte Weiterbildungen der Erfindung ergeben sich aus den Unteransprüchen.

Eine erfindungsgemäße Messvorrichtung zur Erfassung einer Umgebungstemperatur eines Wärme abgebenden oder seiner Umgebung Wärme entziehenden medizinischen Geräts, beispielsweise eines medizinischen Insufflationsgeräts, umfasst ein an einer Außenwand des Geräts angeordnetes oder anordenbares Messgehäuse. Das Messgehäuse ist insbesondere dazu ausgebildet, an der Außenwand des Geräts befestigt zu werden oder in eine Ausnehmung oder einen Durchbruch der Außenwand des Geräts eingesetzt zu werden und kann beispielsweise in Form einer über die Außenwand hinaus ragenden Kappe oder eines Fühlers gestaltet sein. Vorzugsweise ist der Bereich der Außenwand des Geräts, an dem das Messgehäuse angeordnet werden kann, ein Frontbereich bzw. eine Frontplatte des Geräts, da hierdurch in der Regel eine für die Umgebung des Geräts repräsentative Temperatur erfasst werden kann. Das Gerät kann insbesondere ein Wärme abgebendes Gerät sein und Wärmequellen, etwa elektrische Verbraucher, umfassen, deren Verlustwärme beim Betrieb durch Erwärmung der Außenwand des Geräts zu einer Wärmeabgabe des Geräts an seine unmittelbare Umgebung führt. Das Gerät kann aber auch beispielsweise aufgrund der Durchleitung eines Fluids, das kälter als die Umgebung ist, eine kältere Außenwand als die Umgebung aufweisen und dadurch seiner unmittelbaren Umgebung Wärme entziehen. Die erfasste Umgebungstemperatur ist insbesondere eine Lufttemperatur in einer Umgebung des Geräts.

Die erfindungsgemäße Messvorrichtung umfasst weiterhin mindestens einen in dem Messgehäuse aufgenommenen gerätefernen Temperatursensor und mindestens einen in dem Messgehäuse aufgenommenen gerätenahen Temperatursensor. Die Messvorrichtung ist somit derart ausgebildet, dass dann, wenn diese an der Außenwand des Geräts angeordnet ist, der geräteferne Temperatursensor in einem gerätefernen Bereich des Inneren des Messgehäuses angeordnet ist und der mindestens eine gerätenahe Temperatursensor in einem gerätenahen oder auch, wenn das Messgehäuse in die Außenwand des Geräts eingesetzt ist, in einem geräteinneren Bereich des Inneren des Messgehäuses angeordnet ist. Der mindestens eine geräteferne Temperatursensor ist zur Erfassung einer ersten Temperatur vorgesehen und der mindestens eine gerätenahe Temperatursensor zur Erfassung einer zweiten Temperatur, d.h. die Temperatursensoren sind zur Erfassung entsprechender Temperaturwerte ansteuerbar und insbesondere elektrisch entsprechend geschaltet.

Ferner umfasst die erfindungsgemäße Messvorrichtung eine Auswerteeinrichtung, die zur Ermittlung der Umgebungstemperatur aus einer Differenz zwischen den von dem mindestens einen gerätefernen Temperatursensor und dem mindestens einen gerätenahen Temperatursensor erfassten Temperaturen eingerichtet ist.

Neben der Temperaturdifferenz können auch eine oder mehrere der von den Temperatursensoren erfassten Temperaturen selbst bei der Ermittlung der Umgebungstemperatur verwendet werden. Die Auswerteeinrichtung umfasst insbesondere Prozessormittel, etwa einen Mikroprozessor, der zur Ermittlung der Umgebungstemperatur aus der ersten und der zweiten Temperatur unter Verwendung der Differenz zwischen der ersten Temperatur und der zweiten Temperatur programmiert ist. Vorzugsweise ist die Auswerteeinrichtung als Versorgungs- und Auswerteeinrichtung ausgebildet, die die Temperatursensoren mit elektrischer Energie versorgt, zur Temperaturmessung ansteuert und ausliest und die von den Temperatursensoren gelieferten Temperaturwerte wie beschrieben auswertet.

Dadurch, dass die Umgebungstemperatur aus den Temperaturwerten, die von einem gerätefernen und einem gerätenahen Temperatursensor geliefert werden, und unter Verwendung einer Temperaturdifferenz zwischen den Temperaturwerten ermittelt wird, gelingt eine Temperaturerfassung, die weitgehend unabhängig von der von dem Gerät abgegebenen bzw. der von dem Gerät seiner Umgebung entzogenen Wärme ist. Erfindungsgemäß ist erkannt worden, dass durch die Anordnung von mindestens einem gerätenahen und mindestens einem gerätefernen Temperatursensor sowie durch die Auswertung des Temperaturgefälles zwischen den Temperatursensoren eine weitgehend unverfälschte Messung der Umgebungstemperatur möglich ist. Hiermit ist sogar dann noch eine relativ genaue und von der unmittelbaren Umgebung des Geräts weitgehend unbeeinflusste Messung der Umgebungstemperatur möglich, wenn das Gerät in einem Gestell eingebaut ist, in dem sich weitere Wärmequellen befinden und/oder in dem die Luftzirkulation eingeschränkt ist.

In vorteilhafter Weise ist das Messgehäuse aus Kunststoff, insbesondere aus einem Kunststoff mit einer niedrigen Wärmeleitfähigkeit und einer niedrigen Wärmekapazität ausgebildet. Während eine niedrige Wärmeleitfähigkeit eine thermische Entkopplung von dem Gerät ermöglicht, wird durch eine niedrige Wärmekapazität eine rasche Angleichung an die Umgebungstemperatur und damit eine schnelle Erfassung insbesondere von Änderungen der Umgebungstemperatur ermöglicht.

Gemäß einer bevorzugten Ausführungsform der Erfindung ist das Messgehäuse länglich und somit in Form eines Messfühlers oder Temperaturfühlers ausgebildet, der aus der Außenwand des Geräts hervorsteht. Insbesondere ist das Messgehäuse mit einer solchen Länge ausgebildet, dass es, wenn es an oder in die Außenwand des Geräts eingesetzt ist, einen oder wenige Zentimeter, beispielsweise ca. 1,5 cm, nach außen von dieser hervorsteht. Der mindestens eine geräteferne Sensor kann dadurch in einem Bereich des Messgehäuses angeordnet sein, der durch die Oberflächentemperatur des Geräts sowie ggf. durch eine Konvektionsschicht, die sich an der Außenwand des Geräts ausbilden kann, nur wenig beeinflusst wird. Hierdurch kann die Genauigkeit der Erfassung der Umgebungstemperatur verbessert werden.

Vorzugsweise ist das Messgehäuse zumindest in einem Bereich, der für die Anordnung außerhalb des Geräts, d.h. im Außenbereich der Außenwand, vorgesehen ist, geschlossen oder zumindest weitgehend geschlossen ausgebildet. Auch im Innenbereich des Geräts kann das Messgehäuse weitgehend geschlossen sein. Insbesondere kann der Innenraum des Messgehäuses einen geschlossenen oder weitgehend geschlossenen Luftraum bilden. Dadurch, dass das Messgehäuse zumindest im Außenbereich geschlossen ist, kann eine Verschmutzung der Temperatursensoren durch eindringende staubbeladene Luft oder durch eindringende Flüssigkeiten vermieden werden. Ferner wird hierdurch die Reinigung und die Erfüllung der hygienischen Anforderungen bei medizinischen Verwendungen, insbesondere beim Einsatz in einem Operationssaal, erleichtert.

Vorzugsweise ist das Messgehäuse abschnittsweise zylindrisch ausgebildet. Hierdurch wird es erleichtert, das Messgehäuse in eine Außenwand des Geräts einzusetzen, insbesondere in eine in die Außenwand eingebrachte Bohrung. In besonders bevorzugter Weise ist das Messgehäuse abgedichtet in die Bohrung einsetzbar.

Weiterhin ist es vorteilhaft, dass das Messgehäuse im gerätefernen Bereich einen zylindrischen Abschnitt aufweist, der einen kleineren Durchmesser hat als ein zylindrischer Abschnitt des Messgehäuses im gerätenahen Bereich. Während der mit größerem Durchmesser ausgebildete gerätenahe zylindrische Abschnitt ein einfaches und abgedichtetes Einsetzen in eine Bohrung der Außenwand des Geräts ermöglicht sowie eine erhöhte Stabilität gegen ein versehentliches Verbiegen oder Abbrechen des Messgehäuses gewährleistet, ist der mit geringerem Durchmesser ausgebildete geräteferne Bereich vorteilhaft, da der in diesem angeordnete geräteferne Sensor thermisch enger an das Messgehäuse angekoppelt ist. Hierdurch wird die thermische Ankopplung des gerätefernen Temperatursensors an die Umgebungsluft und dadurch die Genauigkeit der Erfassung der Umgebungstemperatur weiter verbessert.

Vorzugsweise sind der mindestens eine geräteferne und/oder der mindestens eine gerätenahe Temperatursensor mit einem Abstand zur Innenseite des Messgehäuses angeordnet und somit insbesondere in einem Luftraum im Inneren des Messgehäuses, besonders bevorzugt näherungsweise in der Mitte des Messgehäuses bzw. bei einem abschnittsweise zylindrischen Messgehäuse nahe an der Zylinderachse. Hierdurch werden eine einfachere Anbringung der Temperatursensoren und eine gegen einseitige Einwirkung von Wärmestrahlung unempfindlichere Temperaturmessung ermöglicht.

Weiterhin ist es bevorzugt, dass die Auswerteeinrichtung zumindest teilweise innerhalb des Messgehäuses, insbesondere in einem geräteinneren Endbereich des Messgehäuses aufgenommen ist. Hierdurch wird ein kompakter Aufbau der Messvorrichtung als Temperaturfühler ermöglicht, wobei die Auswerteeinrichtung derart eingerichtet sein kann, den ermittelten Wert für die Umgebungstemperatur in analoger oder digitaler Form zu liefern. Insbesondere kann die Auswerteeinrichtung derart ausgebildet sein, dass die Messvorrichtung wie ein üblicher Temperatursensor angesteuert und ausgelesen werden kann. Dabei hat sich gezeigt, dass die Ermittlung der Umgebungstemperatur unter Verwendung des Temperaturgefälles zwischen den Temperatursensoren ausreichend robust ist, so dass die Wärmeentwicklung der Auswerteeinrichtung nicht zu einer wesentlichen Verfälschung des ermittelten Temperaturwerts der Umgebungstemperatur führt.

In besonders bevorzugter Weise sind der mindestens eine geräteferne und der mindestens eine gerätenahe Temperatursensor auf einer gemeinsamen, innerhalb des Messgehäuses angeordneten Trägerplatine angebracht. Weiter vorzugsweise kann auch die Auswerteeinrichtung auf derselben Trägerplatine angeordnet sein. Hierdurch werden ein besonders einfacher Aufbau und eine besonders kostengünstige Herstellung der Messvorrichtung ermöglicht.

Gemäß einer bevorzugten Ausführungsform der Erfindung ist die Auswerteeinrichtung derart eingerichtet, dass die Umgebungstemperatur unter Verwendung der Differenz zwischen den von dem mindestens einen gerätefernen Temperatursensor und dem mindestens einen gerätenahen Temperatursensor erfassten Temperaturen und weiter unter Verwendung eines ersten und eines zweiten Wärmewiderstands $R_{th1}$, $R_{th2}$, insbesondere aus dem Quotienten $R_{th1} / R_{th2}$ der Wärmewiderstände, ermittelt wird. Dabei stellt der erste Wärmewiderstand $R_{th1}$ den Wärmewiderstand dar für den Wärmeübergang von der Umgebung des Geräts, insbesondere von der Umgebung eines gerätefernen Bereichs des Messgehäuses, zu dem mindestens einen gerätefernen Temperatursensor und der zweite Wärmewiderstand $R_{th2}$ den Wärmewiderstand für den Wärmeübergang von dem mindestens einen gerätefernen zu dem mindestens einen gerätenahen Temperatursensor. Dadurch, dass die Wärmewiderstände, über die der geräteferne Temperatursensor mit der Umgebung bzw. der gerätenahe mit dem gerätefernen Temperatursensor thermisch gekoppelt ist, bei der Ermittlung der Umgebungstemperatur berücksichtigt werden, wird auf einfache Weise eine genaue Ermittlung der Umgebungstemperatur ermöglicht.

Gemäß einer besonders bevorzugten Ausführungsform der Erfindung ist die Auswerteeinrichtung eingerichtet, um die Umgebungstemperatur $T_a$ gemäß

$$T_a = T_1 + k \, (T_1 - T_2)$$

mit

$$k = R_{th1} / R_{th2}$$

zu ermitteln. Insbesondere weist die Auswerteeinrichtung Prozessormittel auf, die zur Durchführung einer entsprechenden Berechnung programmiert sind. Die Wärmewiderstände $R_{th1}$, Rth2 sind, sobald das Messgehäuse an dem Gerät angebracht ist, insbesondere in eine Bohrung der Außenwand eingesetzt ist, konstant und können bei einer vorangehenden Kalibrierungsmessung ermittelt werden. Eine solche Kalibrierung kann typspezifisch oder gerätespezifisch oder auch nach dem Einsetzen des Messgehäuses an oder in die Außenwand des Geräts individuell durchgeführt werden. Dabei können die Wärmewiderstände in an sich bekannter Weise durch Messung von Temperaturdifferenzen und Wärmeflüssen ermittelt werden, oder es kann der Quotient k der Wärmewiderstände $R_{th1}$, $R_{th2}$ durch Auswertung der von dem mindestens einen gerätefernen und von dem mindestens einen gerätenahen Temperatursensor gelieferten Temperaturwerte unter Verwendung einer von einem externen Temperatursensor ermittelten Umgebungstemperatur berechnet werden. Durch die Erfassung und Auswertung des Temperaturgefälles zwischen dem gerätefernen und dem gerätenahen Temperatursensor unter Berücksichtigung des Quotienten der Wärmewiderstände $R_{th1}$, $R_{th2}$ kann auf einfache Weise und mit hoher Genauigkeit die Ermittlung der Umgebungstemperatur ermöglicht werden.

In bevorzugter Weise sind mindestens zwei geräteferne und/oder mindestens zwei gerätenahe Temperatursensoren vorgesehen, wobei die mindestens zwei gerätefernen Temperatursensoren bzw. die mindestens zwei gerätenahen Temperatursensoren jeweils vorzugsweise zueinander dicht benachbart angeordnet sind, so dass die von den mindestens zwei gerätefernen Temperatursensoren erfassten Temperaturen praktisch gleich sind und die von den mindestens zwei gerätenahen Temperatursensoren erfassten Temperaturen praktisch gleich sind. Insbesondere können die mindestens zwei gerätefernen und/oder die mindestens zwei gerätenahen Temperatursensoren sowie vorzugsweise auch die Auswerteeinrichtung auf einer gemeinsamen, innerhalb des Messgehäuses angeordneten Trägerplatine angebracht sein. Ferner ist die Auswerteeinrichtung vorzugsweise eingerichtet zur Erzeugung eines Fehlersignals, wenn eine Abweichung zwischen den von den mindestens zwei gerätefernen Temperatursensoren erfassten Temperaturen größer als ein vorgebbarer Schwellwert ist bzw. wenn eine Abweichung zwischen den von den mindestens zwei gerätenahen Temperatursensoren erfassten Temperaturen größer als der vorgebbare Schwellwert ist. Aufgrund des Fehlersignals kann beispielsweise ein Benutzer informiert werden oder automatisch eine Maßnahme ergriffen werden, wie etwa eine Heizeinrichtung abgeschaltet werden. Wenn mehr als ein geräteferner bzw. mehr als ein gerätenaher Temperatursensor vorgesehen ist, so kann zur Berechnung der ersten bzw. der zweiten Temperatur jeweils der von einem der Sensoren erfasste Temperaturwert oder auch ein Mittelwert der von mehreren einander benachbarten Sensoren erfassten Temperaturen verwendet werden. Dadurch, dass jeweils mehrere Sensoren zur redundanten Temperaturerfassung vorgesehen sind, wird nicht nur eine Erhöhung der Genauigkeit der Temperaturmessung ermöglicht, sondern es können auch Fehler erkannt und somit die Sicherheit für den Patienten erhöht werden.

Vorzugsweise ist der mindestens eine geräteferne Temperatursensor und/oder der mindestens eine gerätenahe Temperatursensor als Halbleitersensor ausgebildet. Derartige Halbleitersensoren sind an sich bekannt und ermöglichen eine genaue und einfache Erfassung der am Ort des Sensors herrschenden Temperatur.

Gemäß einer alternativen Ausführungsform sind der mindestens eine geräteferne und/oder der mindestens eine gerätenahe Temperatursensor als Infrarotsensor ausgebildet, der die von einer Innenwand des Messgehäuses ausgesandte Wärmestrahlung erfasst. Hierfür sind die Infrarotsensoren auf die Innenseite des Messgehäuses gerichtet. Hiermit ist eine genaue Erfassung der Temperatur des Messgehäuses möglich.

In weiter vorteilhafter Weise ist mindestens ein geräteferner Temperatursensor als Halbleitersensor und mindestens ein geräteferner Temperatursensor als Infrarotsensor ausgebildet und/oder mindestens ein gerätenaher Temperatursensor ist als Halbleitersensor und mindestens ein gerätenaher Temperatursensor als Infrarotsensor ausgebildet. Hierdurch kann eine weiter verbesserte Temperaturerfassung ermöglicht werden.

Bei einem erfindungsgemäßen Messverfahren zur Erfassung einer Umgebungstemperatur eines Wärme abgebenden oder seiner Umgebung Wärme entziehenden medizinischen Geräts wird mit mindestens einem gerätefernen Temperatursensor, der in einem gerätefernen Bereich eines Messgehäuses aufgenommen ist, das an einer Außenwand des Geräts angeordnet ist, eine erste Temperatur gemessen und mit mindestens einem in einem gerätenahen Bereich des Messgehäuses aufgenommenen gerätenahen Temperatursensor eine zweite Temperatur gemessen. Die Umgebungstemperatur wird unter Verwendung einer Differenz zwischen den von dem mindestens einen gerätefernen Temperatursensor und dem mindestens einen gerätenahen Temperatursensor erfassten Temperaturen ermittelt. Weiterhin können die von dem mindestens einen gerätefernen Temperatursensor und dem mindestens einen gerätenahen Temperatursensor erfassten Temperaturen selbst bei der Ermittlung der Umgebungstemperatur verwendet werden. Vorzugsweise wird die Umgebungstemperatur mit einer wie oben beschrieben ausgebildeten Messvorrichtung erfasst, die an der Außenwand des Geräts angeordnet oder in diese eingesetzt ist. Durch ein derartiges Messverfahren ist eine genaue, durch eine Wärmeabgabe oder eine Kühlwirkung des Geräts weitgehend unverfälschte Messung der Umgebungstemperatur möglich.

Gemäß einer bevorzugten Ausführungsform des erfindungsgemäßen Messverfahrens werden außerdem ein erster und ein zweiter Wärmewiderstand zur Ermittlung der Umgebungstemperatur verwendet, wobei der mindestens eine geräteferne Temperatursensor über den ersten Wärmewiderstand mit der Umgebung des Geräts, insbesondere mit der Umgebung eines außerhalb der Außenwand des Geräts angeordneten Bereichs des Messgehäuses, thermisch gekoppelt ist und der mindestens eine gerätenahe Temperatursensor über den zweiten Wärmewiderstand mit dem mindestens einen gerätefernen Temperatursensor thermisch gekoppelt ist. Dabei wird die Umgebungstemperatur $T_a$ gemäß

$$T_a = T_1 + k \, (T_1 - T_2)$$

mit

$$k = R_{th1} \, / \, R_{th2}$$

ermittelt. Die Konstante $k$ oder die beiden Wärmewiderstände $R_{th1}$ und $R_{th2}$ können vorab ermittelt worden sein, beispielsweise bei einer Kalibrierungsmessung, und in einem Speicher zur Verwendung bei der Temperaturermittlung gespeichert sein.

Eine erfindungsgemäße medizinische Insufflationsvorrichtung umfasst ein medizinisches Insufflationsgerät, das eine wie oben beschrieben ausgebildete Messvorrichtung zur Erfassung einer Umgebungstemperatur umfasst oder an dem eine solche angebracht ist. Das Insufflationsgerät ist in an sich bekannter Weise ausgebildet und kann insbesondere Ventile und/oder Pumpen umfassen, um Insufflationsgas, beispielsweise $CO_2$, unter einem geeigneten Druck und ggf. bereits vortemperiert einem Patienten zuzuführen. Weiter umfasst die Insufflationsvorrichtung einen an das Insufflationsgerät anschließbaren Insufflationsschlauch, über den die Zuführung des Insufflationsgases von dem Insufflationsgerät zu einem Insufflationsinstrument erfolgt, etwa zu einer Veress-Nadel, die zur Einleitung des Insufflationsgases in den Patienten dient. Die Insufflationsvorrichtung umfasst ferner eine Heizeinrichtung zur Temperierung des dem Patienten zugeführten Insufflationsgases. Die Heizeinrichtung ist vorzugsweise in den Insufflationsschlauch integriert, wofür dieser mit elektrischen Heizdrähten zur Erwärmung des durch den Schlauch geleiteten Gases ausgestattet sein kann. Die Heizdrähte können einen Anschluss aufweisen, der beim Verbinden des Schlauchs mit dem Insufflationsgerät ebenfalls mit diesem verbunden wird.

Weiterhin umfasst die erfindungsgemäße medizinische Insufflationsvorrichtung eine Steuerungseinrichtung zur Ansteuerung der Heizeinrichtung. Die Steuerungseinrichtung ist dabei derart eingerichtet, dass die Heizeinrichtung in Abhängigkeit von der wie oben beschrieben erfassten Umgebungstemperatur angesteuert wird. Insbesondere steuert oder regelt die Steuerungseinrichtung die Heizleistung der Heizeinrichtung derart, dass unter Berücksichtigung des von der Umgebungstemperatur abhängigen Wärmeübergangs zwischen der Umgebungsluft und dem Insufflationsgas beim Durchströmen des Insufflationsschlauchs auf dem Weg zum Patienten das Insufflationsgas beim Eintritt in den Patienten eine vorgegebene Temperatur hat, die beispielsweise der Körpertemperatur des Patienten entspricht. Wenn das Insufflationsgas bereits im Insufflationsgerät auf die vorgegebene Temperatur erwärmt wird, wird die Heizeinrichtung derart angesteuert, dass das Gas bei der Weiterleitung durch den Schlauch auf dieser Temperatur gehalten wird. Dabei wird die Heizleistung insbesondere in Abhängigkeit von der Gastemperatur am Gasausgang des Insufflationsgeräts, vom Gasdurchfluss und von der Umgebungstemperatur gesteuert. Die Steuerungseinrichtung kann auch zur Steuerung der weiteren Funktionen des Insufflationsgeräts ausgebildet sein, etwa zur Steuerung bzw. Regelung der Durchflusses und des Drucks.

Die wie oben beschrieben erfasste Umgebungstemperatur entspricht in der Regel zumindest näherungsweise der Temperatur in einem Bereich zwischen dem medizinischen Insufflationsgerät und dem Patienten, durch welchen Bereich der Insufflationsschlauch verläuft. Da es von der Umgebungstemperatur abhängt, ob und in welchem Maße sich die Temperatur des Insufflationsgases beim Durchströmen des Insufflationsschlauchs ändert, ist durch Erfassung der Umgebungstemperatur eine sehr genaue Temperierung des Insufflationsgases im Schlauch möglich. Hierdurch kann die Temperatur des in den Patienten eingeleiteten Gases optimal eingestellt werden.

Vorzugsweise umfasst bei einer medizinischen Insufflationsvorrichtung die Messvorrichtung zur Erfassung der Umgebungstemperatur zwei geräteferne und/oder zwei gerätenahe Temperatursensoren, die Messvorrichtung ist zur Erzeugung eines Fehlersignals in dem Fall, dass eine Temperaturdifferenz zwischen zwei benachbarten Sensoren einen Schwellwert überschreitet, ausgebildet, und die Steuerungseinrichtung ist derart eingerichtet, dass aufgrund des Fehlersignals die Heizeinrichtung abgeschaltet wird. Ferner kann in diesem Fall ein Warnsignal an einen Bediener abgegeben werden.

Bei einem erfindungsgemäßen Verfahren zur medizinischen Insufflation wird in an sich bekannter Weise mit einem Insufflationsgerät über einen Insufflationsschlauch ein Insufflationsgas einem Patienten zugeführt, etwa um einen Hohlraum zur Durchführung eines laparoskopischen Eingriffs zu schaffen. Ferner wird wie oben beschrieben eine Umgebungstemperatur des Insufflationsgeräts erfasst und eine Heizeinrichtung in Abhängigkeit von der derart erfassten Umgebungstemperatur angesteuert. Insbesondere wird die Heizeinrichtung mit einer solchen Heizleistung betrieben, dass unter Berücksichtigung des von der Umgebungstemperatur abhängigen Wärmeübergangs zwischen der Umgebungsluft und dem Insufflationsgas beim Durchströmen des Insufflationsschlauchs das Insufflationsgas beim Eintritt in den Patienten eine vorgegebene Temperatur hat. Hierdurch ist eine sehr genaue Einstellung der Temperatur des in den Patienten eingeleiteten Gases möglich. Vorzugsweise wird das erfindungsgemäße Verfahren zur medizinischen Insufflation mit einer wie oben beschrieben ausgebildeten Vorrichtung zur medizinischen Insufflation durchgeführt.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung bevorzugter Ausführungsbeispiele und der beigefügten Zeichnungen. Es zeigen:

    Fig. 1 ein Ausführungsbeispiel einer erfindungsgemäßen Messvorrichtung;
    Fig. 2 ein Ausführungsbeispiel einer erfindungsgemäßen medizinischen Insufflationsvorrichtung mit einer Messvorrichtung gemäß Fig. 1.

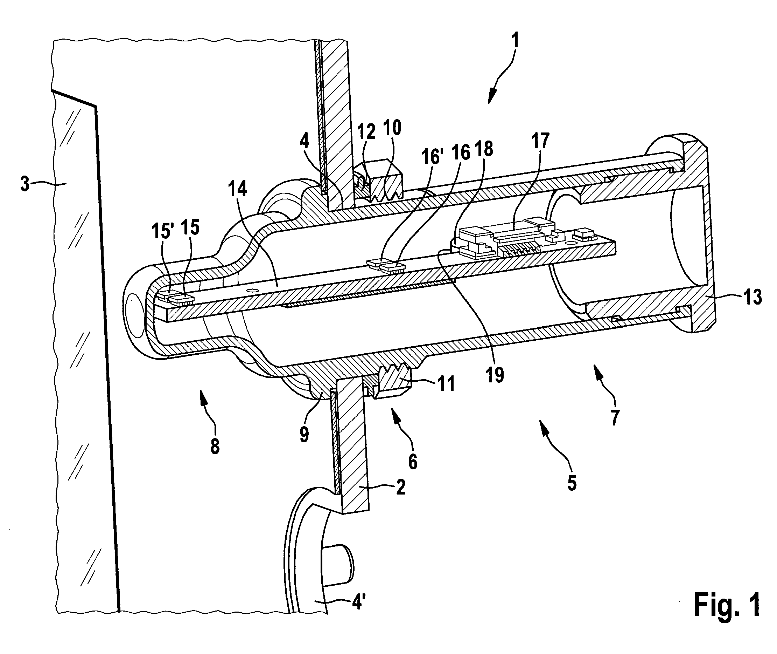

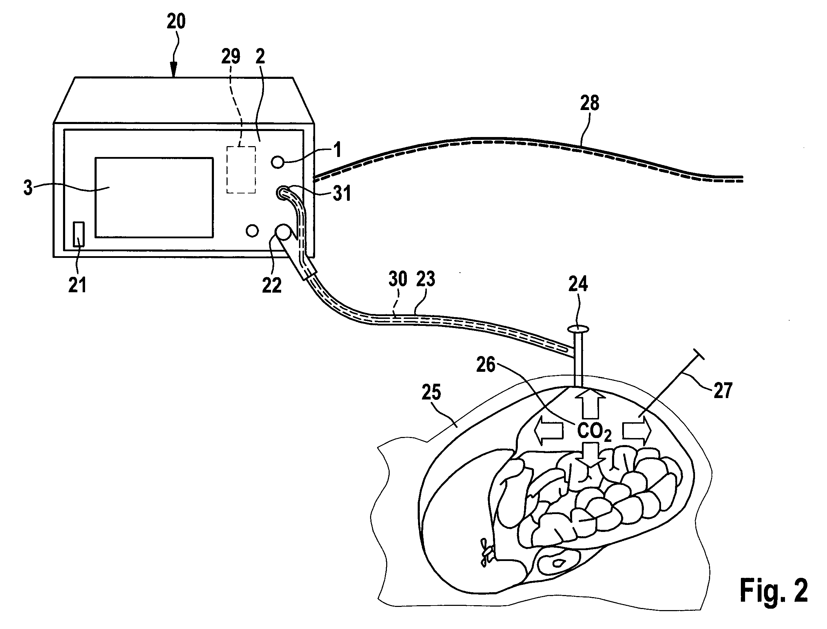

Wie in Fig. 1 gezeigt, ist eine Messvorrichtung zur Erfassung einer Umgebungstemperatur eines Wärme erzeugenden Geräts gemäß einer Ausführungsform der Erfindung als kompakter Temperaturfühler 1 ausgebildet, der in eine Bohrung 4 in einer Frontplatte 2 des Geräts eingesetzt ist. Das Gerät ist beispielsweise ein medizinisches Insufflationsgerät, das wie zu Fig. 2 beschrieben ausgebildet ist und dessen Frontplatte 2 Bedien- und Anzeigeelemente, etwa ein Display 3, aufweist. Im Übrigen ist das Gerätegehäuse in Fig. 1 nicht dargestellt. In Fig. 1 ist der Innenraum des Geräts rechts von der Frontplatte 2 und der Außenraum links von der Frontplatte 2 angeordnet. Die Frontplatte 2 kann weitere Durchbrüche oder Bohrungen 4' zum Einsetzen weiterer Elemente aufweisen. Der Temperaturfühler 1 umfasst ein Messgehäuse 5 mit einem Verbindungsbereich 6 zum Verbinden mit der Frontplatte 2, einem Innenbereich 7, der innerhalb des Geräts angeordnet ist, und einem Außenbereich 8, der außerhalb des Geräts angeordnet ist und der mit seinem gerätefernen Endbereich etwa 15 mm über die Frontplatte 2 hinausragt. Der Verbindungsbereich 6 weist eine Schulter 9 auf, mit der das Messgehäuse 5 von außen an der Frontplatte 2 anliegt, sowie ein Gewinde 10, auf das eine Befestigungsmutter 11 von innen aufgeschraubt wird und über einen Dichtring 12 das Messgehäuse 5 fest in der Bohrung 4 hält. Das Messgehäuse 5 ist aus Kunststoff gefertigt und im Endbereich des Innenbereichs 7 durch einen Kunststoffdeckel 13 abgeschlossen. Der Kunststoffdeckel 13 kann als Normteil verfügbar sein und mit Hilfe einer Schnappverbindung in das Messgehäuse 5 eingesetzt werden. Im Endbereich des Außenbereichs 8 ist das Messgehäuse 5 im Wesentlichen zylindrisch ausgebildet, jedoch mit einem geringeren Durchmesser als im Innenbereich.

Im Inneren des Messgehäuses 5 ist eine in Längsrichtung des Messgehäuses 5 verlaufende Trägerplatine 14 aufgenommen, die in ihrem gerätefernen Endbereich zwei geräteferne Temperatursensoren 15, 15', in ihrem mittleren Bereich zwei gerätenahe Temperatursensoren 16, 16' und in ihrem gerätenahen Endbereich eine Versorgungs- und Auswertungselektronik 17 trägt. Die Temperatursensoren 15, 15', 16, 16' sind jeweils als integrierter Schaltkreis mit einem Halbleitertemperatursensorelement ausgebildet und über einen Bus mit der Versorgungs- und Auswertungselektronik 17 verbunden; derartige Sensoren, die ein digitales Signal liefern, werden beispielsweise von der Fa. Texas Instruments unter der Bezeichnung TMP106 vertrieben. Alternativ oder zusätzlich kann jeweils ein Infrarotsensor vorgesehen sein, der auf die benachbarte Innenwand des Messgehäuses 5 gerichtet ist (nicht dargestellt); für diesen Zweck geeignete Infrarotsensoren werden beispielsweise von der Fa. Texas Instruments unter der Bezeichnung TMP006 vertrieben. Die zwei gerätefernen Temperatursensoren 15, 15' und die zwei gerätenahen Temperatursensoren 16, 16' sind jeweils dicht benachbart zueinander angeordnet, so dass sie die gleiche Temperatur erfassen. Die Versorgungs- und Auswertungselektronik 17 umfasst einen Mikroprozessor sowie Einrichtungen zur Energieversorgung der Temperatursensoren 15, 15', 16, 16'. Über eine Flexplatine 18, die durch einen Durchbruch 19 des Messgehäuses 5 verläuft, ist die Versorgungs- und Auswertungselektronik 17 mit einer Steuerungseinrichtung des Geräts verbunden. Der Durchbruch 19 kann durch eine Vergussmasse oder eine Dichtung geschlossen sein, so dass bei einer entsprechenden Abdichtung des Kunststoffdeckels 13 der Temperaturfühler 1 insgesamt gekapselt sein kann.

Ein derart aufgebauter Temperaturfühler 1 ist kompakt aufgebaut und mit an sich bekannten Verfahren der Kunststoffbearbeitung und Leiterplattenherstellung herstellbar. Das Messgehäuse 5 ist dabei als Kappe ausgebildet, die auf die mit den Temperatursensoren 15, 15', 16, 16' und der Versorgungs- und Auswerteelektronik 17 bestückte Trägerplatine 14 aufgesetzt wird. Der Temperaturfühler 1 wird bei der Montage des Geräts in die Bohrung 4 in der Frontplatte 2 eingesetzt.

Der Mikroprozessor der Versorgungs- und Auswertungselektronik 17 ist zur nachstehend beschriebenen Ermittlung der Umgebungstemperatur $T_a$ des Geräts programmiert. Dabei werden zur Erfassung der Umgebungstemperatur $T_a$ die Temperatursensoren 15, 15', 16, 16' von der Versorgungs- und Auswertungselektronik 17 über den Bus ausgelesen. Aus den von den beiden gerätefernen Temperatursensoren 15, 15' gelieferten Messwerten für die Temperatur werden der Mittelwert $T_1$ und die Differenz $\Delta T_1$ ermittelt. Ebenso werden aus den von den beiden gerätenahen Temperatursensoren 16, 16' gelieferten Temperaturwerten der Mittelwert $T_2$ und die Differenz $\Delta T_2$ bestimmt. Wenn eine der ermittelten Differenzen $\Delta T_1$, $\Delta T_2$ einen Schwellwert $\Delta T_S$, der in Abhängigkeit von der Genauigkeit der Temperatursensoren vorbestimmt ist, überschreitet, wird eine Fehlfunktion angenommen, und die Versorgungs- und Auswertungselektronik 17 sendet über die Flexplatine 18 ein Fehlersignal an eine übergeordnete Steuerungseinrichtung, etwa die Steuerungseinrichtung des medizinischen Insufflationsgeräts, die eine Heizeinrichtung für das Insufflationsgas abschaltet und ein für einen Benutzer wahrnehmbares Warnsignal abgibt. Überschreitet keine der ermittelten Differenzen $\Delta T_1$, $\Delta T_2$ den Schwellwert $\Delta T_S$, so wird die Umgebungstemperatur $T_a$ gemäß

$$T_a = T_1 + k\,(T_1 - T_2)$$

berechnet, wobei k eine Konstante ist, die den Quotienten aus zwei Wärmewiderständen $R_{th1}$, $R_{th2}$ darstellt:

$$k = R_{th1}\,/\,R_{th2}\,.$$

Dabei ist $R_{th1}$ der Wärmewiderstand zwischen der Umgebung und den gerätefernen Temperatursensoren 15, 15', und $R_{th2}$ ist der Wärmewiderstand zwischen den gerätefernen Temperatursensoren 15, 15' und den gerätenahen Temperatursensoren 16, 16'. Beide Wärmewiderstände $R_{th1}$, $R_{th2}$ sind jeweils konstant und von der Anordnung der Temperatursensoren 15, 15', 16, 16', von der Ausbildung des Messgehäuses 5 und der anderen Bauelemente des Temperaturfühlers 1 sowie insbesondere von den verwendeten Materialien abhängig. Für die Wärmewiderstände $R_{th1}$, $R_{th2}$ können Wärmeleitung im Messgehäuse 5 und in der Trägerplatine 14 ebenso wie Wärmeströmung und Wärmestrahlung im Innenraum des Messgehäuses 5 eine Rolle spielen.

Zur Bestimmung der Konstanten k werden vorab die Wärmewiderstände $R_{th1}$, $R_{th2}$ ermittelt oder auch, als Näherungen für $R_{th1}$, $R_{th2}$, die Wärmewiderstände zwischen der Umgebung und dem Außenbereich 8 des Temperaturfühlers 1 sowie zwischen dem Außenbereich 8 und dem Innenbereich 7 des Temperaturfühlers 1. Es kann aber auch durch eine Erfassung von $T_1$ und $T_2$ bei bekannter Umgebungstemperatur $T_a$ direkt die Konstante k bestimmt werden. Eine solche Bestimmung der Konstanten k kann bei einer Kalibrierungsmessung typspezifisch oder individuell für den Temperaturfühler oder auch typspezifisch oder individuell in an dem Gerät montiertem Zustand durchgeführt werden. Es hat sich gezeigt, dass die Wärmewiderstände $R_{th1}$, $R_{th2}$ bzw. die Konstante k ausreichend konstant sind, um eine sehr genaue Messung der Umgebungstemperatur $T_a$ zu ermöglichen.

Wie in Fig. 2 schematisch dargestellt, umfasst eine medizinische Insufflationsvorrichtung gemäß einem Ausführungsbeispiel der Erfindung ein medizinisches Insufflationsgerät 20, dessen Frontplatte 2 Bedien- und Anzeigeelemente aufweist, etwa ein Display 3 und einen Netzschalter 21. In die Frontplatte 2 des Insufflationsgeräts 20 ist ein wie oben beschrieben ausgebildeter Temperaturfühler 1 eingesetzt, der mit seinem gerätefernen Ende etwa 15 mm nach vorne über die Frontplatte 2 hinausragt. Im Inneren des Gehäuses des Insufflationsgeräts 20 sind Ventile und/oder Pumpen sowie eine Steuerungseinrichtung 29 angeordnet, um Insufflationsgas, im dargestellten Beispiel $CO_2$, unter einem geeigneten Druck und ggf. bereits vortemperiert einem Patienten zuzuführen. Weiter umfasst die Insufflationsvorrichtung einen an einen Anschluss 22 angeschlossenen Insufflationsschlauch 23, über den die Zuführung des Insufflationsgases von dem Insufflationsgerät 20 zu einem Insufflationsinstrument erfolgt, etwa zu einer Veress-Nadel 24, die die Bauchdecke 25 des Patienten durchsticht. Durch die Veress-Nadel 24 wird $CO_2$ in die Bauchhöhle 26 des Patienten eingeleitet, wie durch die Blockpfeile angedeutet, um einen ausreichenden Hohlraum für die Durchführung eines endoskopischen Eingriffs zu schaffen. In Fig. 2 sind symbolisch weitere endoskopische Instrumente 27 angedeutet, die bei dem unter endoskopischer Sicht durchgeführten Eingriff verwendet werden. In den Insufflationsschlauch 23 ist eine Heizeinrichtung in Form eines Heizdrahts 30 integriert, der innerhalb des Insufflationsschlauchs 23 gestreckt oder auch in Form einer Heizwendel verlaufen kann; hierdurch kann das dem Patienten zugeführte Insufflationsgas erwärmt werden. Der Heizdraht 30 ist mit einem entsprechenden elektrischen Anschluss 31 des Insufflationsgeräts 20 verbunden.

Die Steuerungseinrichtung ist auch zur Steuerung der weiteren Funktionen des Insufflationsgeräts 20 ausgebildet, etwa zur Steuerung bzw. Regelung der Durchflusses und des Drucks. Die Bedienung des Insufflationsgeräts erfolgt über das als Touch-Screen ausgebildete Display 3. In Fig. 2 ist symbolisch ein Zuleitungsschlauch 28 zur Zuführung des Insufflationsgases von einer nicht dargestellten externen Gasversorgung zum Insufflationsgerät 20 angedeutet.

Mit Hilfe des Temperaturfühlers 1 wird wie oben beschrieben die Umgebungstemperatur $T_a$ des Insufflationsgeräts 20 erfasst, die die Temperatur eines Raumbereichs vor der Frontplatte 2 des Geräts ist und die gleich oder zumindest repräsentativ für die Temperatur in einem Raumbereich zwischen dem Insufflationsgerät 20 und dem Patienten ist. Durch diesen Bereich verläuft der Insufflationsschlauch 23. Die Steuerungseinrichtung steuert die Heizeinrichtung in Abhängigkeit von der erfassten Umgebungstemperatur $T_a$ an, so dass unter Berücksichtigung des von dieser abhängigen Wärmeübergangs zwischen der Umgebungsluft und dem Insufflationsgas beim Durchströmen des Insufflationsschlauchs 23 auf dem Weg zum Patienten das Insufflationsgas beim Eintritt in die Bauchhöhle 26 des Patienten eine vorgegebene Temperatur hat, beispielsweise nahezu die Körpertemperatur des Patienten. Da es von der Umgebungstemperatur $T_a$ abhängt, ob und in welchem Maße sich die Temperatur des Insufflationsgases beim Durchströmen des Insufflationsschlauchs 23 ändert, ist auf diese Weise eine sehr genaue Temperierung des in die Bauchhöhle 26 des Patienten eingeleiteten Gases möglich.

Bezugszeichenliste

| | |
|---|---|
| 1 | Temperaturfühler |
| 2 | Frontplatte |
| 3 | Display |
| 4, 4' | Bohrung |
| 5 | Messgehäuse |
| 6 | Verbindungsbereich |
| 7 | Innenbereich |
| 8 | Außenbereich |
| 9 | Schulter |
| 10 | Gewinde |
| 11 | Befestigungsmutter |
| 12 | Dichtring |
| 13 | Kunststoffdeckel |
| 14 | Trägerplatine |
| 15, 15' | Temperatursensor |
| 16, 16' | Temperatursensor |
| 17 | Versorgungs- und Auswertungselektronik |
| 18 | Flexplatine |
| 19 | Durchbruch |
| 20 | Insufflationsgerät |
| 21 | Netzschalter |
| 22 | Anschluss |
| 23 | Insufflationsschlauch |
| 24 | Veress-Nadel |
| 25 | Bauchdecke |
| 26 | Bauchhöhle |
| 27 | Instrument |
| 28 | Zuleitungsschlauch |
| 29 | Steuerungseinrichtung |
| 30 | Heizdraht |
| 31 | Anschluss |

**Patentansprüche**

1. Messvorrichtung zur Erfassung einer Umgebungstemperatur $T_a$ eines Wärme abgebenden oder seiner Umgebung Wärme entziehenden medizinischen Geräts, mit einem an einer Außenwand des Geräts anordnenbaren Messgehäuse (5), in dem mindestens ein

geräteferner Temperatursensor (15, 15') und mindestens ein gerätenaher Temperatursensor (16, 16') aufgenommen sind, wobei der mindestens eine geräteferne Temperatursensor (15, 15') eine erste

Temperatur $T_1$ und der mindestens eine gerätenahe Temperatursensor (16, 16') eine zweite Temperatur $T_2$ erfassen, und mit einer Auswerteeinrichtung, die zur Ermittlung der Umgebungstemperatur $T_a$ unter Verwendung einer Differenz zwischen der von dem mindestens einen gerätefernen Temperatursensor (15, 15') erfassten ersten Temperatur $T_1$ und der von dem mindestens einen gerätenahen Temperatursensor (16, 16') erfassten zweiten Temperatur $T_2$ eingerichtet ist.

2. Messvorrichtung nach Anspruch 1, **dadurch gekennzeichnet, dass** das Messgehäuse (5), wenn es an oder in die Außenwand des Geräts eingesetzt ist, länglich in Form eines über die Außenwand des Geräts hinausragenden Temperaturfühlers ausgebildet ist.

3. Messvorrichtung nach Anspruch 1 oder 2, **dadurch gekennzeichnet, dass** das Messgehäuse (5) zumindest in einem Außenbereich (8), der außerhalb des Geräts anordenbar ist, geschlossen ist.

4. Messvorrichtung nach Anspruch 3, **dadurch gekennzeichnet, dass** das Messgehäuse (5) im Außenbereich (8) einen zylindrischen Abschnitt aufweist, der einen kleineren Durchmesser als ein zylindrischer Abschnitt eines Innenbereichs (7), der innerhalb des Gerätes anordenbar ist, aufweist.

5. Messvorrichtung nach einem der vorhergehenden Ansprüche, **dadurch gekennzeichnet, dass** der mindestens eine geräteferne Temperatursensor (15, 15') und/oder der mindestens eine gerätenahe Temperatursensor (16, 16') in einem Abstand zur Innenseite des Messgehäuses (5) angeordnet sind.

6. Messvorrichtung nach einem der vorhergehenden Ansprüche, **dadurch gekennzeichnet, dass** die Auswerteeinrichtung zumindest teilweise in dem Messgehäuse (5) aufgenommen ist.

7. Messvorrichtung nach einem der vorhergehenden Ansprüche, **dadurch gekennzeichnet, dass** der mindestens eine geräteferne Temperatursensor (15, 15') und der mindestens eine gerätenahe Temperatursensor (16, 16') sowie vorzugsweise die Auswerteeinrichtung auf einer gemeinsamen, in dem Messgehäuse (5) aufgenommenen Trägerplatine (14) angeordnet sind.

8. Messvorrichtung nach einem der vorhergehenden Ansprüche, **dadurch gekennzeichnet, dass** die Auswerteeinrichtung zur Ermittlung der Umgebungstemperatur $T_a$ unter Verwendung eines Wärmewiderstands $R_{th1}$ zwischen der Umgebung des Geräts und dem mindestens einen gerätefernen Temperatursensor (15, 15') und eines Wärmewiderstands $R_{th2}$ zwischen dem mindestens einen gerätefernen Temperatursensor (15, 15') und dem mindestens einen gerätenahen Temperatursensor (16, 16') einrichtbar ist.

9. Messvorrichtung nach Anspruch 8, **dadurch gekennzeichnet, dass** die Auswerteeinrichtung eingerichtet ist zur Ermittlung der Umgebungstemperatur $T_a$ gemäß

$$T_a = T_1 + k\,(T_1 - T_2)$$

mit

$$k = R_{th1} / R_{th2} \,.$$

10. Messvorrichtung nach einem der vorhergehenden Ansprüche, **dadurch gekennzeichnet, dass** die Messvorrichtung mindestens zwei geräteferne Temperatursensoren (15, 15') und/oder mindestens zwei gerätenahe Temperatursensoren (16, 16') aufweist, und dass die Auswerteeinrichtung zur Erzeugung eines Fehlersignals in dem Fall, dass eine Abweichung $\Delta T_1$ zwischen den von den mindestens zwei gerätefernen Temperatursensoren (15, 15') erfassten Temperaturen und/oder eine Abweichung $\Delta T_2$ zwischen den von den mindestens zwei gerätenahen Temperatursensoren (16, 16') erfassten Temperaturen größer als ein vorgebbarer Schwellwert $\Delta T_S$ ist, eingerichtet ist.

11. Messvorrichtung nach einem der vorhergehenden Ansprüche, **dadurch gekennzeichnet, dass** der mindestens eine gerätenahe Temperatursensor (16, 16') und der mindestens eine geräteferne Temperatursensor (15, 15') als Halbleitersensoren ausgebildet sind oder dass der mindestens eine gerätenahe Temperatursensor (16, 16') und der mindestens eine geräteferne Temperatursensor (15, 15') als Infrarotsensoren ausgebildet sind oder dass mindestens ein gerätenaher Temperatursensor (16) als Halbleitersensor und mindestens ein gerätenaher Temperatursensor (16') als Infrarotsensor und/oder mindestens ein geräteferner Temperatursensor (15) als Halbleitersensor und mindestens ein geräteferner Temperatursensor (15') als Infrarotsensor ausgebildet sind.

12. Messverfahren zur Erfassung einer Umgebungstemperatur $T_a$ eines Wärme abgebenden oder seiner Umgebung Wärme entziehenden medizinischen Geräts, wobei mit mindestens einem in einem gerätefernen Bereich eines an einer Außenwand des Geräts angeordneten Messgehäuses (5) angeordneten gerätefernen Temperatursensor (15, 15') eine

erste Temperatur $T_1$ und mit mindestens einem in einem gerätenahen Bereich des Messgehäuses (5) angeordneten gerätenahen Temperatursensor (16, 16') eine zweite Temperatur $T_2$ gemessen werden, eine Temperaturdifferenz $(T_1 - T_2)$ ermittelt wird und wobei die Umgebungstemperatur $T_a$ unter Verwendung der Temperaturdifferenz $(T_1 - T_2)$ ermittelt wird.

13. Messverfahren nach Anspruch 12, **dadurch gekennzeichnet, dass** die Umgebungstemperatur $T_a$ gemäß

$$T_a = T_1 + k\,(T_1 - T_2)$$

mit

$$k = R_{th1} / R_{th2}$$

ermittelt wird, wobei k oder $R_{th1}$ und $R_{th2}$ vorab ermittelt worden sind.

14. Vorrichtung zur medizinischen Insufflation, umfassend ein medizinisches Insufflationsgerät (20), einen Insufflationsschlauch (23) zur Zuführung eines Insufflationsgases vom Insufflationsgerät (20) zu einem Patienten, eine Heizeinrichtung zur Temperierung des dem Patienten zugeführten Insufflationsgases und eine Steuerungseinrichtung zur Ansteuerung der Heizeinrichtung, **dadurch gekennzeichnet, dass** das Insufflationsgerät (20) eine Messvorrichtung zur Erfassung einer Umgebungstemperatur $T_a$ gemäß einem der Ansprüche 1 bis 11 aufweist und dass die Steuerungseinrichtung zur Ansteuerung der Heizeinrichtung unter Berücksichtigung der erfassten Umgebungstemperatur $T_a$ eingerichtet ist.

15. Vorrichtung nach Anspruch 14, **dadurch gekennzeichnet, dass** zur Erfassung der Umgebungstemperatur $T_a$ mindestens, zwei geräteferne Temperatursensoren (15, 15') und/oder mindestens zwei gerätenahe Temperatursensoren (16, 16') vorhanden sind, dass die Auswerteeinrichtung zur Ermittlung einer Temperaturdifferenz $\Delta T_1$, $\Delta T_2$ zwischen zwei gerätefernen Temperatursensoren (15, 15') und/oder zwischen zwei gerätenahen Temperatursensoren (16, 16') und zur Erzeugung eines Fehlersignals in dem Fall, dass mindestens eine Temperaturdifferenz $\Delta T_1$, $\Delta T_2$ einen vorgebbaren Schwellwert $\Delta T_S$ überschreitet, eingerichtet ist und dass die Steuerungseinrichtung derart eingerichtet ist, dass aufgrund des Fehlersignals die Heizeinrichtung abgeschaltet wird.

16. Verfahren zur medizinischen Insufflation, wobei mit einem Insufflationsgerät (20) über einen Insufflationsschlauch (23) ein Insufflationsgas einem Patienten zugeführt wird, das mit einer Heizeinrichtung temperiert wird, **dadurch gekennzeichnet, dass** eine Umgebungstemperatur $T_a$ des Insufflationsgeräts (20) mit einem Messverfahren gemäß Anspruch 12 oder 13 erfasst wird und die Heizeinrichtung in Abhängigkeit von der erfassten Umgebungstemperatur $T_a$ angesteuert wird.

**Claims**

1. Measuring apparatus for detecting an ambient temperature $T_a$ of a heat-emitting medical machine or medical machine which withdraws heat from the surroundings thereof, comprising a measurement housing (5) which is arrangeable at an outer wall of the machine, with at least one machine-distant temperature sensor (15, 15') and at least one machine-proximate temperature sensor (16, 16') being received in said measurement housing, wherein the at least one machine-distant temperature sensor (15, 15') detects a first temperature $T_1$ and the at least one machine-proximate temperature sensor (16, 16') detects a second temperature $T_2$, and comprising an evaluation device which is configured to ascertain the ambient temperature $T_a$ using a difference between the first temperature $T_1$ detected by the at least one machine-distant temperature sensor (15, 15') and the second temperature $T_2$ detected by the at least one machine-proximate temperature sensor (16, 16').

2. Measuring apparatus according to Claim 1, **characterized in that** the measurement housing (5), when it is placed on, or inserted into, the outer wall of the machine, has an elongate embodiment in the form of a temperature sensor projecting out of the outer wall of the machine.

3. Measuring apparatus according to Claim 1 or 2, **characterized in that** the measurement housing (5) is closed at least in one outer region (8), which is arrangeable outside of the machine.

4. Measuring apparatus according to Claim 3, **characterized in that** the measurement housing (5) has a cylindrical portion in the outer region (8), said cylindrical portion having a smaller diameter than a cylindrical portion of an inner region (7), which is arrangeable within the machine.

5. Measuring apparatus according to one of the preceding claims, **characterized in that** the at least one machine-distant temperature sensor (15, 15') and/or the at least one machine-proximate temperature sensor (16, 16') are arranged at a distance from the inner side of the measurement housing (5).

6. Measuring apparatus according to one of the preceding claims, **characterized in that** the evaluation device is at least partly received in the measurement housing (5).

7. Measuring apparatus according to one of the preceding claims, **characterized in that** the at least one machine-distant temperature sensor (15, 15') and the at least one machine-proximate temperature sensor (16, 16') and, preferably, the evaluation device are arranged on a common carrying printed circuit board (14) received in the measurement housing (5).

8. Measuring apparatus according to one of the preceding claims, **characterized in that** the evaluation device is configurable to determine the ambient temperature $T_a$ using a thermal resistance $R_{th1}$ between the surroundings of the machine and the at least one machine-distant temperature sensor (15, 15') and a thermal resistance $R_{th2}$ between the at least one machine-distant temperature sensor (15, 15') and the at least one machine-proximate temperature sensor (16, 16').

9. Measuring apparatus according to Claim 8, **characterized in that** the evaluation device is configured to determine the ambient temperature $T_a$ in accordance with

$$T_a = T_1 + k(T_1 - T_2)$$

where

$$k = R_{th1}/R_{th2}.$$

10. Measuring apparatus according to one of the preceding claims, **characterized in that** the measuring apparatus has at least two machine-distant temperature sensors (15, 15') and/or at least two machine-proximate temperature sensors (16, 16') and **in that** the evaluation device is configured to produce an error signal in the case where a deviation $\Delta T_1$ between the temperatures detected by the at least two machine-distant temperature sensors (15, 15') and/or a deviation $\Delta T_2$ between the temperatures detected by the at least two machine-proximate temperature sensors (16, 16') is greater than a predeterminable threshold $\Delta T_S$.

11. Measuring apparatus according to one of the preceding claims, **characterized in that** the at least one machine-proximate temperature sensor (16, 16') and the at least one machine-distant temperature sensor (15, 15') are embodied as semiconductor sensors or **in that** the at least one machine-proximate temperature sensor (16, 16') and the at least one machine-distant temperature sensor (15, 15') are embodied as infrared sensors or **in that** at least one machine-proximate temperature sensor (16) is embodied as a semiconductor sensor and at least one machine-proximate temperature sensor (16') is embodied as an infrared sensor and/or at least one machine-distant temperature sensor (15) is embodied as a semiconductor sensor and at least one machine-distant temperature sensor (15') is embodied as an infrared sensor.

12. Measuring method for detecting an ambient temperature $T_a$ of a heat-emitting medical machine or medical machine which withdraws heat from the surroundings thereof, wherein a first temperature $T_1$ is detected by means of at least one machine-distant temperature sensor (15, 15') arranged in a machine-distant region of a measurement housing (5) arranged on the outer wall of the machine and a second temperature $T_2$ is detected by means of at least one machine-proximate temperature sensor (16, 16') arranged in a machine-proximate region of the measurement housing (5), a temperature difference ($T_1 - T_2$) is ascertained and wherein the ambient temperature $T_a$ is determined using the temperature difference ($T_1 - T_2$).

13. Measuring method according to Claim 12, **characterized in that** the ambient temperature $T_a$ is established in accordance with

$$T_a = T_1 + k(T_1 - T_2)$$

where

$$k = R_{th1}/R_{th2},$$

wherein k or $R_{th1}$ and $R_{th2}$ is/are established in advance.

14. Apparatus for medical insufflation, comprising a medical insufflation machine (20), an insufflation tube (23) for supplying insufflation gas from the insufflation machine (20) to a patient, a heating device for temperature-control of the insufflation gas supplied to the patient and a control device for actuating the heating device, **characterized in that** the insufflation machine (20) has a measuring apparatus for detecting an ambient temperature $T_a$ according to one of Claims 1 to 11 and **in that** the control device is configured to actuate the heating device taking account of the detected ambient temperature $T_a$.

**15.** Apparatus according to Claim 14, **characterized in that** at least two machine-distant temperature sensors (15, 15') and/or at least two machine-proximate temperature sensors (16, 16') are present for detecting the ambient temperature $T_a$, **in that** the evaluation device is configured to establish a temperature difference $\Delta T_1$, $\Delta T_2$ between two machine-distant temperature sensors (15, 15') and/or between two machine-proximate temperature sensors (16, 16') and to produce an error signal in the case where at least one temperature difference $\Delta T_1$, $\Delta T_2$ exceeds a predetermined threshold $\Delta T_S$, and **in that** the control device is configured in such a way that the heating device is switched off due to the error signal.

**16.** Method for medical insufflation, wherein an insufflation machine (20) is used to supply insufflation gas to a patient by means of an insufflation tube (23), said insufflation gas being under temperature control by a heating device, **characterized in that** an ambient temperature $T_a$ of the insufflation machine (20) is detected by a measuring method according to Claim 12 or 13 and the heating device is actuated in a manner dependent on the detected ambient temperature $T_a$.

## Revendications

**1.** Dispositif de mesure destiné à détecter une température ambiante $T_a$ d'un appareillage médical restituant de la chaleur ou prélevant de la chaleur dans son environnement, avec un boîtier de mesure (5) susceptible d'être placé sur une paroi extérieure de l'appareillage, dans lequel sont logés au moins un capteur de température (15, 15') éloigné de l'appareillage et au moins un capteur de température (16, 16') proche de l'appareillage, l'au moins un capteur de température (15, 15') éloigné de l'appareillage détectant une première température $T_1$ et l'au moins un capteur de température (16, 16') proche de l'appareillage détectant une deuxième température $T_2$ et avec un système d'évaluation qui est aménagé pour déterminer la température ambiante $T_a$ en utilisant une différence entre la première température $T_1$ détectée par l'au moins un capteur de température (15, 15') éloigné de l'appareillage et la deuxième température $T_2$ détectée par l'au moins un capteur de température (16, 16') proche de l'appareillage.

**2.** Dispositif de mesure selon la revendication 1, **caractérisé en ce que** lorsqu'il est inséré sur ou dans la paroi latérale de l'appareillage, le boîtier de mesure (5) est conçu en étant allongé, sous la forme d'une sonde thermique saillant pardessus l'appareillage.

**3.** Dispositif de mesure selon la revendication 1 ou la revendication 2, **caractérisé en ce que** le boîtier de mesure (5) est fermé dans au moins une zone extérieure (8) à l'extérieur de l'appareillage.

**4.** Dispositif de mesure selon la revendication 3, caractérisé en ce dans la zone extérieure (8), le boîtier de mesure (5) comporte une partie cylindrique qui présente un diamètre inférieur à celui d'une partie cylindrique d'une zone intérieure (7) qui est susceptible d'être placée à l'intérieur de l'appareillage.

**5.** Dispositif de mesure selon l'une quelconque des revendications précédentes, **caractérisé en ce que** l'au moins un capteur de température (15, 15') éloigné de l'appareillage et/ou l'au moins un capteur de température (16, 16') proche de l'appareillage sont placés avec un écart par rapport la face intérieure du boîtier de mesure (5).

**6.** Dispositif de mesure selon l'une quelconque des revendications précédentes, **caractérisé en ce que** le système d'évaluation est logé au moins en partie dans le boîtier de mesure (5).

**7.** Dispositif de mesure selon l'une quelconque des revendications précédentes, **caractérisé en ce que** l'au moins un capteur de température (15, 15') éloigné de l'appareillage et l'au moins un capteur de température (16, 16') proche de l'appareillage, ainsi que de préférence le système d'évaluation sont placés sur une carte support (14) logée dans le boîtier de mesure (5).

**8.** Dispositif de mesure selon l'une quelconque des revendications précédentes, **caractérisé en ce que** le système d'évaluation est susceptible d'être aménage pour déterminer la température ambiante $T_a$, en utilisant une résistance thermique $R_{th1}$ entre l'environnement de l'appareillage et l'au moins un capteur de température (15, 15') éloigné de l'appareillage et une résistance thermique $R_{th2}$ entre l'au moins un capteur de température (15, 15') éloigné de l'appareillage et l'au moins un capteur de température (16, 16') proche de l'appareillage.

**9.** Dispositif de mesure selon la revendication 8, **caractérisé en ce que** le système d'évaluation est aménagé pour déterminer la température ambiante $T_a$ selon

$$T_a = T_1 + k \ (T_1 - T_2)$$

sachant que

$$k = R_{th1} \ / \ R_{th2}$$

**10.** Dispositif de mesure selon l'une quelconque des revendications précédentes, **caractérisé en ce que** le dispositif comporte au moins deux capteurs de température (15, 15') éloignés de l'appareillage et/ou au moins deux capteurs de température (16, 16') proches de l'appareillage et **en ce que** le système dévaluation est aménagé pour délivrer un signal de défaut dans le cas où une différence $\Delta T_1$ entre les températures détectées par les au moins deux capteurs de température (15, 15') éloignés de l'appareillage et/ou une différence $\Delta T_2$ entre les températures détectées par les au moins deux capteurs de température (16, 16') proches de l'appareillage est supérieure à une valeur seuil $\Delta T_S$ prédéfinissable.

**11.** Dispositif de mesure selon l'une quelconque des revendications précédentes, **caractérisé en ce que** l'au moins un capteur de température (16, 16') proche de l'appareillage et l'au moins un capteur de température (15, 15') éloigné de l'appareillage sont conçus en tant que capteurs à semi-conducteur ou **en ce que** l'au moins un capteur de température (16, 16') proche de l'appareillage et l'au moins un capteur de température (15, 15') éloigné de l'appareillage sont conçus en tant que capteurs à infrarouge ou **en ce qu'**au moins un capteur de température (16) proche de l'appareillage est conçu en tant que capteur à semi-conducteur et au moins un capteur de température (16') proche de l'appareillage est conçu en tant que capteur à infrarouge et/ou au moins un capteur de température (15) éloigné de l'appareillage est conçu en tant que capteur à semi-conducteur et au moins un capteur de température (15') éloigné de l'appareillage est conçu en tant que capteur à infrarouge.

**12.** Procédé de mesure destiné à détecter une température ambiante $T_a$ d'un appareillage médical restituant de la chaleur ou prélevant de la chaleur dans son environnement, à l'aide d'au moins un capteur de température (15, 15') éloigné de l'appareillage placé dans une zone éloignée de l'appareillage d'un boîtier de mesure (5) placé sur une paroi extérieure de l'appareillage étant mesurée une première température $T_1$ et avec au moins un capteur de température (16, 16') proche de l'appareillage placé dans une zone proche de l'appareillage du boîtier de mesure (5) étant mesurée une deuxième température $T_2$, une température différentielle. $(T_1 - T_2)$ étant déterminée et la température ambiante $T_a$ étant déterminée, en utilisant la température différentielle $(T_1 - T_2)$.

**13.** Procédé de mesure selon la revendication 12, **caractérisé en ce que** la température ambiant $T_a$ est déterminée selon

$$T_a = T_1 + k\ (T_1 - T_2)$$

sachant que

$$k = R_{th1}\ /\ R_{th2},$$

k ou $R_{th1}$ et $R_{th2}$ ayant été déterminés au préalable.

**14.** Dispositif pour l'insufflation médicale, comprenant un appareillage insufflateur (20), un flexible d'insufflation (23) destiné à alimenter un gaz à insuffler de l'appareillage insufflateur (20) vers un patient, un système de chauffage destiné à tempérer le gaz à insuffler alimenté vers le patient et un système de commande destiné à amorcer le système de chauffage, **caractérisé en ce que** l'appareillage insufflateur (20) comporte un dispositif de mesure destiné à détecter une température ambiante $T_a$ selon l'une quelconque des revendications 1 à 11 et **en ce que** le système de commande est aménagé pour amorcer le système de chauffage sous considération de la température ambiante $T_a$ détectée.

**15.** Dispositif selon la revendication 14, **caractérisé en ce que** pour détecter la température ambiante $T_a$, au moins deux capteurs de température (15, 15') éloignés de l'appareillage et/ou au moins deux capteurs de température (16, 16') proches de l'appareillage sont présents, **en ce que** l'unité d'évaluation est aménagée pour déterminer une température différentielle $\Delta T_1$, $\Delta T_2$ entre deux capteurs de température (15, 15') éloignés de l'appareillage et/ou entre deux capteurs de température (16, 16') proches de l'appareillage et pour générer un signal de défauts dans le cas où au moins une température différentielle $\Delta T_1$, $\Delta T_2$ dépasse une valeur seuil $\Delta T_S$ prédéfinissable et **en ce que** le système de commande est aménagé de sorte que le système de chauffage soit mis à l'arrêt suite à un signal de défauts.

**16.** Procédé destiné à l'insufflation médicale, à l'aide d'un appareillage insufflateur (20), par l'intermédiaire d'un flexible d'insufflation (23) étant alimenté vers un patient un gaz d'insufflation qui est tempéré à l'aide d'un système de chauffage, **caractérisé en ce qu'**une température ambiante $T_a$ de l'appareil insufflateur (20) est détectée à l'aide d'un procédé de mesure selon la revendication 12 ou la revendication 13 et **en ce que** le système de chauffage est amorcé en fonction de la température ambiante $T_a$ détectée.

Fig. 1

EP 2 916 119 B1

Fig. 2

EP 2 916 119 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0564953 A1
- DE 102006019402 A1
- DE 69223723 T2
- DE 102005049676 B3
- WO 2010023255 A1
- DE 19800753 A1

- DD 87677 B1
- GB 2131175 A
- US 2012277673 A1
- US 20050209813 A1
- US 4741476 A